(19) Europäisches Patentamt
European Patent Office
Office européen des brevets

(11) Veröffentlichungsnummer: **0 271 501 B1**

(12) **EUROPÄISCHE PATENTSCHRIFT**

(45) Veröffentlichungstag der Patentschrift:
27.02.91 Patentblatt 91/09

(51) Int. Cl.⁵: **A61F 2/44**

(21) Anmeldenummer: **87902440.4**

(22) Anmeldetag: **14.05.87**

(86) Internationale Anmeldenummer:
**PCT/DE87/00224**

(87) Internationale Veröffentlichungsnummer:
**WO 87/07827 30.12.87 Gazette 87/29**

(54) **IMPLANTAT ZUM FIXIEREN BENACHBARTER WIRBELKNOCHEN DER WIRBELSÄULE.**

(30) Priorität: **19.06.86 DE 3620549**

(43) Veröffentlichungstag der Anmeldung:
**22.06.88 Patentblatt 88/25**

(45) Bekanntmachung des Hinweises auf die
Patenterteilung:
**27.02.91 Patentblatt 91/09**

(84) Benannte Vertragsstaaten:
**AT BE CH DE FR GB IT LI LU NL SE**

(56) Entgegenhaltungen:
**EP-A- 0 042 271
DE-A- 2 910 627
US-A- 4 501 269**

(73) Patentinhaber: **S + G IMPLANTS GMBH
Grapengiesserstrasse 34
D-2400 Lübeck (DE)**

(72) Erfinder: **GRUNDEI, Hans
Gärtnergasse 4
D-2400 Lübeck (DE)**
Erfinder: **THOMAS, Wolfram
Poppenbütteler Landstrasse 12
D-2000 Hamburg (DE)**

(74) Vertreter: **Wilcken, Hugo, Dr. et al
Patentanwälte Dr. Hugo Wilcken Dipl.-Ing.
Thomas Wilcken Musterbahn 1
D-2400 Lübeck (DE)**

**Beschreibung**

Die Erfindung bezieht sich auf ein Implantat zum Fixieren benachbarter Wirbelknochen der Wirbelsäule, die an den sich gegenüberliegenden Flächen zur Bildung eines Aufnahmeraumes für das mit einem Werkzeug einzutreibende Implantat teilweise abgetragen sind, wobei das Implantat offerzellig und metallisch ist. Ein derartiges Implantat geht aus der DE-A-29 10627 als bekannt hervor.

Es ist bekannt, bei Schäden an der Wirbelsäule zwei benachbarte Wirbelknochen mittels Platten und Schrauben starr zu verbinden, um eine sogenannte Osteosynthese herzustellen. Dabei ist es jedoch erforderlich, die Platten und Schrauben nach bestimmter Zeit wieder zu entfernen, was einen erneuten operativen Eingriff erfordert.

Es ist weiter bekannt, die einander zugekehrten Flächen zweier benachbarter Wirbelknochen teilweise auszuräumen und in den dadurch geschaffenen Aufnahmeraum Knochenmaterial einzutreiben, welches vorher von anderen Knochen des Patienten entnommen werden muß.

Die Aufgabe der Erfindung besteht darin, zwei benachbarte Wirbelknochen der Wirbelsäule durch ein bleibendes Implantat starr zu verbinden, ohne daß das Eintreiber das Implantates in die Wirbelsäule zu Schäden des Patienten fürbren kann.

Diese Aufgabe wird nach der Erfindung bei dem eingangs erwähnten Implantat dadurch gelöst, daß zumindest das proximale, die Werkzeugangriffsfläche bildende Ende des im übrigen offenzelligen, zylindrischen oder rohrförmigen Implantates massiv ausgebildet ist.

In ein solches offenzelliges Implantat kann in bekannter Weise Knochengewebe mit anschließender Knochenzellenbildung einwachsen und dadurch eine Einheit mit den Knochen bilden. Ein solches offenzelliges Implantat muß von der Bauchhöhle aus nach seitlichem Wegschieben der Eingeweide mit einem Hammer oder Schlagwerkzeug in den geschaffenen Aufnahmeraum der benachbarten Wirbelknochen eingetrieben werden, und dies ist nur dann möglich, wenn die Angriffsfläche des Implantates für das Schlagwerkzeug massiv ausgebildet ist, den anderenfalls könnten beim Eintreiben Zellenwandteile des Implantates absplittern und dann zu Schäden für den Patienten führen.

Das Implantat ist als Zylinder oder vorteilhaft als Kegelstumpf durchgehend offenzellig auszubilden oder auch zur Gewichtseinsparung als offenzelliger Rohrkörper auszubilden, der die Außenform eines Kegelstumpfes besitzt, dessen Neigungswinkel klein ist und etwa zwischen 4 bis 8° beträgt. Die Wanddicke des Rohrkörpers beträgt etwa 3 bis 6 mm und das dem Angriff des Schlagwerkzeuges zugekehrte proximale, den größeren Durchmesser besitzende Ende des metallischen Implantates oder auch beide Enden sind vollwandig massiv auszubilden. Dies kann dadurch z.B. erreicht werden, däß das eine oder beide Enden des offenzelligen, metallischen Implantates aus einem vollwandigen metallischen Ring besteht bzw. bestehen, der bzw. die mit dem offenzelligen Implantatkörper durch Verschweißung verbunden ist bzw. sind.

Die Erfindung wird nachstehend anhand der Zeichnung im einzelnen erläutert. Es zeigen :

Figur 1 einen zylindrischen oder kegelstumpfförmigen Implantatkörper mit unterbrochenem Längsschnitt und einer Stirnansicht,

Figur 2 einen rohrförmigen Implantatkörper in Konusstumpfform,

Figur 3 die Lage des Implantates zwischen zwei benachbarten, miteinander starr zu verbin-denden Wirbelknochen,

Figur 4 eine teilweise innere Ansicht der Wirbelsäule mit einem fixierenden Implantat,

Figur 5 eine teilweise Seitenansicht der Wirbelsäule mit Teilschnitt durch zwei benachbarte, durch das Implantat fixierte Wirbelknochen.

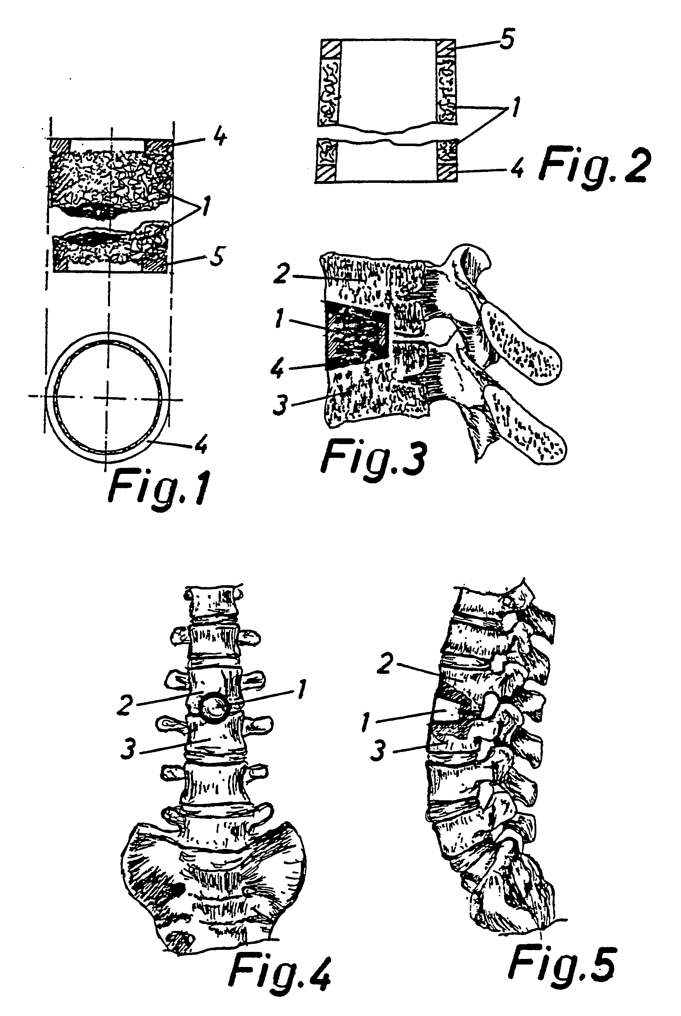

Das Implantat 1 besteht aus einem offenzelligen, metallischen Körper in Form eines Zylinders nach Figur 1 oder eines Rohres nach Figur 2, wobei der Körper ein vorteilhaft konusförmig mit einem Neigungswinkel zwischen 4 bis 8° und mit einer Wanddicke des rohrförmigen Implantates zwischen 3 bis 6 mm ausgebildet ist.

Das mit einem Hammer oder einem Schlagwerkzeug in einen durch vorheriges Abtragen hergestellten Aufnahmeraum an den zugekehrten Flächen zweier benachbarter Wirbelknochen 2 und 3 einzutreibende Implantat 1 ist an einem proximalen Ende 4, an das das Schlagwerkzeug angreift, vollwandig ausgebildet. Vorteilhaft ist es, das andere, distale Ende ebenfalls vollwandig auszubilden, um das Absplittern von kleinen Metallstücken der offenen Implantatszellen zu vermeiden.

Die vollwandigen Enden 4 und 5 des Implantates 1 können aus metallischen Ringen bestehen, die mit dem offenzelligen, metallischen Implantatkörper 1 verschweißt sind.

**Ansprüche**

1. Implantat zum Fixieren benachbarter Wirbelknochen der Wirbelsäule, die an den sich gegenüberliegenden Flächen zur Bildung eines Aufnahmeraumes für das mit einem Werkzeug einzutreibende Implantat teilweise abgetragen sind, wobei das Implantat offenzellig und metallisch ist, dadurch gekennzeichnet, daß zumindest das proximale, die Werkzeugangriffsfläche bildende Ende (4) des im übrigen offenzelligen, zylindrischen oder rohrförmigen Implantates (1) massiv ausgebildet ist.

2. Implantat nach Anspruch 1, dadurch gekennzeichnet, daß die Wanddicke des rohrförmigen Implantates 3 bis 6 mm beträgt.

3. Implantat nach Anspruch 1 oder 2, dadurch gekennzeichnet daß seine äußere Kontur anstelle der zylindrischen Form Kegelstumpfes hat, die sich zum der Angriffsfläche abgekehrten Ende hin konisch verjüngt.

4. Implantat nach einem der Ansprüche 1 bis 3, dadurch gekennzeichnet, daß mindestens das proximale massive Ende (4) des Rohrkörpers (1) aus einem massiven, mit dem offenzelligen Rohrkörper (1) verbundenen Metallring besteht.

5. Implantat nach Anspruch 3 oder 4, dadurch gekennzeichnet, daß der Neigungswinkel des Kegelstumpfes zwischen 4 bis 8° liegt.

## Claims

1. Implant for securing neighbouring vertebrae of the spinal column which are partially worn away on the opposing surfaces for forming a receiving space for the implant to be driven in using a tool, the implant being open-cell and metallic, characterised in that at least the proximal end (4), which forms the tool operative surface, of the otherwise open-cell, cylindrical or tubular implant (1) has a solid construction.

2. Implant according to claim 1, characterised in that the wall thickness of the tubular implant is 3 to 6mm.

3. Implant according to claim 1 or claim 2, characterised in that its outer contour has the shape of a frustum which is tapered conically towards the end facing away from the operative surface, instead of the cylindrical shape.

4. Implant according to any of claims 1 to 3, characterised in that at least the proximal solid end (4) of the tubular body (1) comprises a solid metal ring connected to the open-cell tubular body (1).

5. Implant according to claim 3 or claim 4, characterised in that the angle of inclination of the frustum is 4 to 8 degrees.

## Revendications

1. Implant destiné à fixer des vertèbres voisines de la colonne vertébale, qui sont partiellement décalées sur les surfaces tournées l'une vers l'autre pour créer un espace de réception pour l'implant à introduire à l'aide d'un outil, l'implant étant métallique, à cellule ouverte, caractérisé en ce qu'au moins la partie proximale, qui constitue l'extrémité (4), constituant la surface d'attaque de l'outil, de l'implant (1) qui pour le reste est en cellule ouverte, cylindrique ou tubulaire, est de structure massive.

2. Implant selon la revendication 1, caractérisé en ce que l'épaisseur de paroi de l'implant tubulaire est de 3 à 6 mm.

3. Implant selon la revendication 1 ou 2, caractérisé en ce que son contour extérieur présente, au lieu de la forme cylindrique la forme d'un tronc de cône, qui se rétrécit coniquement en direction de l'extrémité opposée à la surface d'attaque.

4. Implant selon l'une des revendications 1 à 3, caractérisé en ce qu'au moins l'extrémité proximale massive (4) du corps tubulaire (1) se compose d'un anneau métallique massif, relié au corps tubulaire à cellule ouverte (1).

5. Implant selon la revendication 3 ou 4, caractérisé en ce que l'angle d'inclinaison du tronc de cône est compris entre 4 et 8°.

EP 0 271 501 B1

Fig.1

Fig.2

Fig.3

Fig.4

Fig.5

4